# EUROPEAN PATENT SPECIFICATION

(11) **EP 1 980 279 B1**
(45) Date of publication and mention of the grant of the patent: **16.11.2011**
(21) Application number: 08154327.4
(22) Date of filing: 10.04.2008
(51) Int. Cl.: A61L 31/12, A61L 31/14

(54) **A medical device**
Medizinische Vorrichtung
Dispositif médical

(30) Priority: 12.04.2007 FI 20075246
(43) Date of publication of application: 15.10.2008
(73) Proprietor: BIORETEC OY, 33720 Tampere (FI)
(72) Inventor: Törmälä, Pertti, 33300, Tampere (FI); Huttunen, Mikko, 33230, TAMPERE (FI); Heino, Harri, 33340, Tampere (FI)
(74) Representative: Hakola, Unto Tapani

(56) References cited:
- EP-A- 1 493 404
- WO-A-2006/114483
- HUTTUNEN ET AL: "Fibre reinforced bioresorbable composites for spinal surgery" ACTA BIOMATERIALIA, ELSEVIER, AMSTERDAM, NL, vol. 2, no. 5, 1 September 2006 (2006-09-01), pages 575-587, XP005596260 ISSN: 1742-7061

## Description

### FIELD OF THE INVENTION

The present invention relates to a medical device.

### BACKGROUND OF THE INVENTION

Biostable or bioabsorbable devices are used in surgery for musculoskeletal applications, such as e.g. (a) screws, plates, pins, tacks or nails for the fixation of bone fractures and/or osteotomies to immobilize the bone fragments for healing, (b) suture anchors, tacks, screws, bolts, nails, clamps and other devices for soft tissue-to-bone (or -into-bone) and soft tissue-to-soft tissue fixation, or (c) cervical wedges and lumbar cages and plates and screws for vertebral interbody fusion and other operations in spinal surgery.

Most biostable devices are typically made of metallic alloys (see e.g. M. E. Muller, M. Allgöwer, R. Schneider, H. Willenegger "Manual of Internal Fixation", Springer-Verlag, Berlin Heidelberg New York 1979). However, there are several disadvantages in the use of metallic implants. One such disadvantage is bone resorption caused by high modulus bone plates and screws, which carry most of the external loads, leading to stress shielding produced by the modulus mismatch between metals and bone. Another disadvantage is the possibility of corrosion. Therefore, it is recommended that surgeons should remove metallic devices (like bone plates and screws) in a second operation once the fracture has healed. Another possible drawback of certain metals is that they can interfere with magnetic imaging (MRI) and with computer tomography (CT), making post surgical assessment of the healing process more difficult.

When considering applications in spinal fusion surgery, metallic implants in the interbody spinal fusion application have proven to be effective, but the high strength of metals may lead to further problems, such as stress shielding, reduction of blood supply at the implantation site and the possibility of corrosion wear and debris formation. The high strength of metals may also increase the risk of implant subsidence into vertebrae. Therefore, materials whose mechanical properties are closer to those of bone tissue are needed.

Bioabsorbable polymeric fracture fixation devices have been developed and studied as replacements for metallic implants (see e.g. S. Vainionpää, P. Rokkanen, P. Törmälä, "Surgical Applications of Biodegradable Polymers in Human Tissue", Progress in Polymer Science, Vol. 14, 1989, pp. 679-716). The advantages of these devices are that the materials are resorbed in the body and the degradation products exit via metabolic routes. Hence, a second operation is not required. Additionally, the strength properties of the bioabsorbable polymeric devices decreases when the device degrades, and hence the bone is progressively loaded more and more, which promotes bone regeneration (according to Wolff's law).

One limitation of many prior art bioabsorbable materials and devices is their relatively low strength. In the case of cortical bone fractures, for example, non-reinforced poly lactic acid (PLLA) plates and screws are initially too weak to permit patient mobilization (see e.g. J. Eitenmüller, K.L. Gerlach, T. Schmickal, H. Krause, "An in Vivo Evaluation of a New High Molecular Weight Polylactide Osteosynthesis Device", European Congress on Biomaterials, Bologna Italy, September 14-17, 1986, p. 94).

Non-reinforced polylactic acid devices typically have three-point bending strengths of 50-100 MPa and modulus of 3.5-4.0 GPa, and particulate reinforced (hydroxyapatite) polylactic acid devices have values of 25-30 MPa and 5.0 GPa, respectively.

Composites of poly-L-lactide and β-tricalcium phosphate are more fragile than pure polymeric implants if not reinforced by any technique. An example of this is the first generation of ACL screws composed of composites of poly-L-lactide and β-tricalcium phosphate which yielded unfavourable results, as these screws tend to break during the implantation. See Smith CA, Tennent TD, Pearson SE, Beach WR. Fracture of Bilok interference screws on insertion during anterior cruciate ligament reconstruction. Arthroscopy. 2003 Nov;19(9):E115-17.

We developed earlier self-reinforced bioresorbable polymeric composites to improve the strength of bioresorbable polymer devices. These show relatively good mechanical properties: e.g., bending strength of 360±70 MPa and bending modulus of 12±2 GPa, respectively, have been reported (see P. Törmälä, "Biodegradable Self-Reinforced Composite Materials; Manufacturing, Structure and Mechanical Properties", Clinical Materials, Vol. 10, 1992, pp. 29-34). Self-reinforcing is a good option when increased mechanical properties are needed in the longitudinal direction of the implant, but it may not be feasible if increased mechanical properties are needed in the circumferential direction of the exterior surface of the implant.

One option to increase mechanical properties of bioabsorbable polymeric devices is to use fiber reinforcement. A. Saikku-Backström et al. studied in vivo and in vitro hydrolysis of poly- 96L/4D-lactide fiber reinforced poly-96L/4D-lactide matrix (fibrillated SR-PLA96) rods (diam. 1.1 mm). The rods had much higher initial strength properties than non-reinforced materials reported in the literature. After 168 days (24 weeks) of in vitro hydrolysis in buffered saline at 37°C, the bending strength was still 86.7% (195 MPa) of the initial value. It can be concluded that these bioabsorbable polylactide fiber reinforced polylactide rods had a good strength retention in hydrolytic conditions, but these composites did not contain a bioactive agent. See: A. Saikku- Backström et al. in J. Mater. Sci: Mater. Med. W (1999) p. 1-8.

When the composite is made of three components: polymer matrix, reinforcing polymer fibers and ceramic particulate filler, the mechanical properties can be improved (M. Kellomaki et al., 13th Eur. Conf. Biom., Abstracts, Gothenburg, Sweden, Sept. 4-7, 1997, p. 90). These composites are, however, composed of laminated layers. Therefore, a partial fracture, such as delamination and fragment migration, is a risk in clinical applications.

The use of ceramic fibers as a reinforcement has also been reported. Zimmerman et al. developed unidirectional composites of poly-L-lactide matrix reinforced with calcium / phosphorous oxide (CaP) based biodegradable glass fibers. This composite showed good initial strength properties, but the strength reinforcing effect of the CaP fibers in hydrolytic conditions (in vitro: a tris-buffered saline of pH 7.4 at 37°C) was lost totally after 23 days of immersion, while only 35% of the initial strength and 45% of the initial modulus was retained. It can be concluded that this composite, which was reinforced with long ceramic fibers, lost its strength too rapidly to be applied as a raw material for bone fracture fixation devices. See M. Zimmerman, T. Guastavin, J. R. Parsons, H. Alexander and T.C. Lin: "The in vivo biocompatibility and in vitro degradation of absorbable glass fiber reinforced composites", 12th Ann. Meeting Soc. Biomater., p. 16, Minneapolis-St.Paul, Minnesota, USA (1986).

A common property of most polymeric implants is the lack of bony ongrowth on the materials. In contrast, such bone apposition is produced by bioactive, osteoconductive ceramics, such as bioactive glasses (see e.g. O. H. Andersson, K.H. Karlsson, "Bioactive Glass, Biomaterials Today and Tomorrow", Proceedings of the Finnish Dental Society Days of Research, Tampere, Finland, 10-11 November 1995, Gillot Oy, Turku, 1996, pp. 15-16). By adding (compounding) bioactive particulate filler or short fiber ceramics, such as bioactive glasses or calcium phosphate ceramics into polymers to produce composites, the bioactivity of the polymeric material can be improved. This effect has been demonstrated e.g. in dental composites and in bone cement (see e.g. J. C. Behiri, M. Braden, S.N. Khorashani, D. Wiwattanadate, W. Bonfield, "Advanced Bone Cement for Long Term Orthopaedic Applications", Bioceramics; Vol. 4, ed. W. Bonfield, G.W. Hastings and K.E. Tanner, Butterworth-Heinemann Ltd., Oxford, 1991 , pp. 301-307).

Bioactive composites of calcium phosphate ceramics and polylactides have proven to be an effective alternative to plain polymeric materials in certain applications. For example latest generations of bioabsorbable ACL fixation screws contain bioactive ceramic particulate components in bioabsorbable polymeric matrices (e.g. The Matryx^{™} Interference Screw, ConMed Linvatec, The Bio-INTRAFIX System, DePuy/Mitek/Johnson & Johnson, The BIOCRYL Interference Screw, DePuy/Mitek/Johnson & Johnson).

Frank Kandziora et al studied bioabsorbable composite cervical fusion cages (PCC) composed of 50% calcium phosphate and 50% polylactide, and compared them to the iliac crest auto grafts and bioabsorbable polymeric PLDLA 70/30 cages in a sheep model. After 12 weeks, there was no significant difference between the bioabsorbable PLDLLA 70/30 cage and the tricortical bone graft. Although six of eight PCC cages developed cracks after only 12 weeks, this bioabsorbable composite cage showed significantly better distractive properties, significantly higher biomechanical stiffness, and an advanced interbody fusion in comparison with the tricortical iliac crest bone graft. In conclusion, bioabsorbable composite cages gave better results than iliac crest autografts and PLDLLA cages, but as a remarkable pitfall, cracks were formed (i.e. implant failure) in the implant structure during the healing phase. See. F. Kandziora, R. Pflugmacher, M. Scholz, T. Eindorf, K.J. Schnake, and N.P. Haas, Bioabsorbable Interbody Cages in a Sheep Cervical Spine Fusion Model, SPINE 2004 Volume 29, Number 17, pp 1845-1855.

According to a study by Frank Kandziora et al, fusion implants composed of polylactide and calcium phosphate gave a better outcome than plain bioabsorbable polymer in an animal model. The implant failure (cracks) was, however, a remarkable pitfall and risk.

Bioabsorbable composites of hydroxyapatite and copolymers of polyhydroxybutyrate and polyhydroxyvalerate have been described by C. Doyle, K.E. Tanner, W. Bonfield, see "In Vitro and in Vivo Evaluation of Polyhydroxybutyrate and of Polyhydroxyvalerate Reinforced with Hydroxyapatite", Biomaterials, Vol. 12, 1991 , pp. 841-847). The main limitation of these bioabsorbable composites is their inadequate mechanical strength for extensive bone fracture fixation. Also, the use of hydroxyapatite and polylactic acid composites has been reported. See Y. Ikada, H. H. Suong, Y. Shimizu, S. Watanabe, T. Nakamura, M. Suzuki, AT. Shimamoto, "Osteosynthetic Pin", U.S. Patent 4,898,186, 1990.

Prior art also teaches biodegradable and bioactive composites with at least one resorbable polymeric reinforcing element and at least one ceramic reinforcing element with a particle size between 2 µm and 150 µm (see P. Törmälä, M. Kellomäki, W. Bonfield, K.E. Tanner, "Bioactive and Biodegradable Composites of Polymers and Ceramics or Glasses and Method to Manufacture such Composites", EP 1 009 448 B1). The geometry of the final product may need the machining of the composite to the form of a final product, which can cause breaking of the fibers, leading to the weakening of the implant material and to initiation points for crack propagation.

Q.-Q. Qin et al. describe in WO 2004049904 a flexible, bioactive mesh comprising glass fibers and first resorbable polymer fibers in which said glass fibers are interwoven with said first resorbable polymer fibers. However, this is a flexible, low modulus material because there is no matrix polymer which could transfer loads from fibers to each other and could prevent fibers from moving in relation to each other when the mesh is subjected to external forces.

J. D. Gresser et al described in US 6548002 B2 a compression molding technique for interbody spinal fusion devices, which are 25 to 100% bioabsorbable and contain bioabsorbable reinforcing fibers. In that technique, a bioactive filler was used and the reinforcing fibers were under tension in the mold.

Keith D'Alessio et al described in US 5674286 A a manufacturing technique for completely bioabsorbable fiber reinforced composite materials. That invention was related to polymeric matrix fibers and polymeric reinforcing fibers being different in their thermal behavior. The adhesion between the reinforcing element and the matrix polymer was achieved under increased pressure and at a processing temperature between the glass transition temperature of the polymeric matrix fibers and the melting point of the polymeric reinforcing fibers.

Thomas H. Barrows described in US 6511748 B1 a method for manufacturing bioabsorbable fiber reinforced composites, which can also contain mineral filler, such as hydroxyl apatite particles. US 6511748 B1 is, however, related to bioabsorbable fibers, comprising a semicrystalline fiber-forming core polymer and an amorphous sheath polymer, wherein the core polymer and the sheath polymer are separately melt extruded and connected to one another through an adhesive bond. In US 6511748 B1, the preferred manufacturing method was injection molding where the fiber reinforcement was in the form of short chopped 1-10 mm fibers comprising 10 to 70% of the volume of the matrix. Alternatively, the injection molding cavity could have been loaded with bioabsorbable fiber reinforcement, which is wrapped around the mandrel that serves as a core of an injection molding cavity.

WO 2006114483 describes fiber reinforced bioabsorbable and bioactive composites where both polymeric and ceramic fiber reinforcement was used in the composite structure, which gave composites superior mechanical properties having a modulus in the range of that of cortical bone, especially in the beginning of the degradation process as described in WO2006114483.

### BRIEF SUMMARY OF THE INVENTION

There exists a need for strong bioabsorbable composite materials and devices with high strength to guarantee the safe initial consolidation and healing of bone fractures. There exists further a need for such materials and devices which additionally retain the high strength values under hydrolytic conditions at 37°C over several weeks to guarantee the safe consolidation and healing of bone fractures and to guarantee that possible breaking of the composite device will not lead to migration of implant fragments into the surrounding tissues. The latter is a concern especially in implantation sites with a high risk of further damage by implant fragmentation, such as that in the spine surgery. There exist further needs for such materials, which are additionally osteoconductive, which means that they promote and facilitate bone healing.

There also exists a need for such devices which do not crack or split during implantation. Such devices are for example bioabsorbable screws which are inserted into a drill hole in a bone.

The medical device described in this application comprises a body which comprises bioabsorbable basic material. The body has a longitudinal axis and it comprises an inner region and a peripheral region transverse to the longitudinal axis. The cross-section of the body may have different shapes and the area of the cross-section may vary in the longitudinal direction of the body. The peripheral region surrounds the inner region. The inner region is made of bioabsorbable basic material. The peripheral region also comprises bioabsorbable basic material, but in addition to the bioabsorbable basic material it comprises a bioabsorbable reinforcing structure wound around the inner region.

The body consists of a core and the bioabsorbable reinforcing structure. The structures, the materials and the manufacturing methods of the core and the bioabsorbable reinforcing structure will be described in detail below.

The bioabsorbable basic material refers to the material of the core which will be described below. The bioabsorbable reinforcing structure may be any suitable structure described below, but often it is a monofilament fiber which is wound around the core one or more times. If the monofilament fiber is wound around the core several times, it may advance spirally around the core. The inner region is completely made of the bioabsorbable basic material but the peripheral region also comprises the bioabsorbable reinforcing structure.

### DETAILED DESCRIPTION OF THE INVENTION

Composite materials with continuous fiber reinforcement surrounding at least one exterior surface of the device are feasible in the manufacture of e.g. bone fracture fixation devices, because fiber reinforcement will improve their mechanical properties and increase their safety if implant failure occurs during the healing phase, and therefore, they will lead to improved healing and to a lower risk of damage if the implant fails during the healing phase. The high strength of the implant guarantees the safe progress of healing after the early consolidation of the fracture.

The present invention relates to bioabsorbable and bioactive composite materials and medical devices for surgical musculoskeletal applications, the materials and devices comprising a core of a polymeric matrix , with bioactive filler, whose outer surface is reinforced at least partly with a bioabsorbable structure. The bioabsorbable structure may contain continuous, bioabsorbable polymeric fiber(s) and optionally with additional bioactive, bioabsorbable ceramic or glass fiber(s). The bioabsorbable fiber reinforcement of this invention is continuous and composed of long fiber(s), which is (are) located on at least one exterior surface of the core billet. The continuous fiber reinforcement may form a continuous circumferential loop-like structure on or close to at least one exterior surface of the composite, optionally continuing also into the interior of the composite structure. Bioactivity of the device is achieved (a) by using bioactive ceramic particles or short fibers which are mixed with bioabsorbable polymer matrix, and (b) by using bioactive ceramic or glass fibers in combination with polymeric fiber reinforcement to form the circumferential long fiber loop-reinforcement.

The bioabsorbable structure comprises one or more long fibers. The long fiber refers in this application to a fiber whose length exceeds or is equal to the length of the circumference of the core. The long fibers may be continuous filaments forming continuous multifilament yarns or fiber bundles. The long fiber may also be a single monofilament fiber. The long fiber may also be a textile structure. For example, a yarn may be spun of staple fibers, and the resulting yarn may be used as such, or manufactured to, for example, a braid, a knitted or a woven fabric. The same definition applies both to the bioabsorbable polymeric fibers, the ceramic fibers and the bioactive glass fibers.

The core is a three-dimensional body which has an outer wall. The outer wall extends in the longitudinal direction of the core. The outer wall ends at end walls. The core may be, for example, a cylindrical body whose casing forms the outer wall, and the circular walls, which are perpendicular to the longitudinal axis, form the end walls. However, there are a lot of possible variations concerning the shapes of the outer and end walls because those shapes vary depending on the specific application. The core may be a solid body, or it may contain cavities or holes for different purposes.

The bioabsorbable and fiber reinforced composites of this invention can be used to manufacture medical implants for musculoskeletal surgery where the breakage of the implant material is a concern during or after the implantation, as in ACL ligament reconstruction with bioabsorbable screws, or during the healing phase, when applying vertebral interbody fusion implants in spinal fusion operations, and in the load bearing applications when using pins and screws in bone fracture fixations.

We have surprisingly found that bioabsorbable bioactive composites can be reinforced using continuous bioabsorbable fiber reinforcement on at least one of the composite's exterior surfaces. The main function of a continuous fiber reinforcement circulating around the implant material is to increase its strength and safety in applications where possible migrating fragments in case of an implant break could cause severe damage, e.g. in spine surgery. Thus, said continuous fiber reinforcement circulating around the composite's exterior surface can increase patient safety in the healing phase after surgical intervention. Alternatively, the continuous bioabsorbable reinforcement is useful in applications involving a risk that the medical device, such as a screw or a pin, crack or split during implantation.

Composites reinforced by continuous fibers circulating around the composite's exterior surface described in this invention have improved mechanical properties compared to non-reinforced devices, because the reinforcement will change the fracturing mechanism of the material and increase its mechanical properties. Even though breakage of the implant material may occur, continuous reinforcing fibers will hold together the fragmented parts and prevent their migration into the surrounding tissues. Therefore, the continuous fiber reinforced implants of this invention are more reliable under loading than reinforced implants of prior art.

The manufacturing of medical implants, such as spacers (e.g. wedges) for cervical spine fusion, from reinforced materials of prior art is possible, but in such cases creating the final shape of the implant may lead to non continuous fibers in the implant structure, especially on its exterior surfaces, because the implant may require a specific geometry including holes or cavities for an implantation instrument.

According to one advantageous embodiment of this invention, at least some of the reinforcement fibers retain their strength longer than the matrix. Such fibers surround the matrix material, preventing migration of matrix particles during their late fragmentation. This is important in applications where implant fragmentation and migration could cause severe damage, such as paraplegia in the spinal fusion applications.

According to another advantageous embodiment of this invention, the reinforcement fibers lose their strength before or simultaneously with the matrix. This behaviour is advantageous in applications in which extra strength is only required during implantation but the extra strength is insignificant after the implantation. Such applications include, for example, implants which are surrounded by a healing bone. In other words, there is no risk that parts of the medical device could escape from the implantation site of the medical device.

Accordingly, this invention describes composite materials and devices, which comprise at least one polymeric matrix phase (core), at least one bioactive ceramic phase (filler and/or reinforcing fibers) embedded therein to make the core osteoconductive, and at least one bioabsorbable polymeric reinforcing long fiber phase wound around the core.

The outer reinforcing long fiber phase may also contain long ceramic or glass fibers to make also this outer phase osteoconductive. The core may also contain porosity to facilitate new bone growth therein.

The reinforced composite materials and devices described in this invention have a better combination of mechanical properties and osteoconductivity when compared to the reinforced and non-reinforced materials and devices of prior art. Outer reinforcement of the core with continuous slowly degrading polymeric fibers surrounding the core on at least on of its exterior surfaces, will increase both the load bearing capacity retention and the safety of the implant, while the fiber reinforcement wound around the core has preferably a longer strength retention time than the matrix and therefore the fibers have the capability to bind possible fragments of the core material if core fragmentation occurs. At the same time, the implant expresses good biocompatibility, while the implant surfaces which are not covered by reinforcing circumferential surface fibers, have a high concentration of osteoconductive, bioactive glass or ceramic particles, which are advantageously in a close contact with the surrounding bone. Osteoconductive ceramic particles can, however, also be present on the exterior surface where the reinforcing fibers are located, if the core billet has a specific geometry which includes grooves for fibers. Consequently, new bone tissue can grow rapidly in contact with the osteoconductive surfaces and inside them, especially when the bioabsorption of the polymer matrix and ceramic or glass filler or fibers proceeds. Additional porosity in the matrix can facilitate new bone formation inside the core material. If the bioactive ceramic or glass fibers can be in combination with polymeric long fibers circumferential on the outer surface of the core, also this outer surface will be osteoconductive, facilitating new bone formation also on this outer surface. Stiff ceramic or glass fibers also increase the stiffness of the implant, especially in the early phase of the healing period.

It is important that the relative amounts of the different components of the implant of the innovation can be controlled accurately (the amounts of matrix polymer, its porosity, ceramic or glass filler or fibers, outer reinforcing long polymer fibers and optional outer reinforcing long ceramic or bioactive glass fibers). This is important, because the ratio of the components will affect both the mechanical properties and the osteoconductivity of the material and the device.

The bioabsorbable matrix polymer (or polymers) of this invention may be chosen so that it has a shorter strength retention time *in vivo* than at least part of the continuous outer polymeric long fiber reinforcement (or reinforcements) has. Consequently, at least some of the polymeric long fibers will have a longer strength retention time *in vivo* (in tissue environment) than the core has.

The core is composed of polymer (or polymers) and bioactive glass or ceramic filler. Those components will be premixed (compounded) together using techniques of polymer technology, such as mechanical mixing, melt flow extrusion, or injection molding.

The polymeric long fiber reinforcement can be manufactured from the raw materials using traditional fiber forming techniques, such as melt spinning, wet spinning or dry spinning.

The core can be processed mechanically or by using melt flow techniques, such as injection molding or transfer molding, into the desired form needed for further processing, such as compression molding, to produce the final product (device). If the melt flow technique is used, then the raw materials of the core can be also mixed together during that process or they can be premixed using melt flow techniques such as extrusion.

Continuous polymeric long fiber reinforcements can be used as fibers composed of one polymer or polymer alloy, as fiber bundles composed of several fiber elements of at least one polymer, or as prefabricated products, such as braids, knitted or woven fabrics, manufactured by means of methods of textile technology.

There are several technical possibilities to introduce the polymeric long fiber reinforcement in the final structure of the composite device to form a continuous and circular fiber reinforcement on the surface of the device. To name a few, these include, e.g., compression molding, filament winding or pultrusion. For example, when the compression molding technique is used, the manufacturing method of the medical device comprises first the manufacturing of the core of bioabsorbable material. After that, the core may be provided with grooves, but that is not absolutely necessary. In the next step, the bioabsorbable structure, such as a monofilament, is wound around the core. The core with the bioabsorbable structure around the core is treated in a mold under pressure so that the shape of the body is achieved.

The outer long fiber reinforcement can be composed of at least one polymer component or of both polymeric long fiber and ceramic long fiber components.

Bioabsorbable polymeric long fibers used as reinforcement and possible reinforcing ceramic fibers differ significantly from each other in their mechanical behavior. Polymeric long fibers are tough and strong, and they can thus increase the toughness and strength values, such as the tensile, bending, tear, and impact strength of the composites. Ceramic long fibers have high stiffness, and they can thus increase the stiffness (modulus values) of even polymer fiber reinforced composites.

By combining long bioabsorbable polymeric fibers and long ceramic fibers in different ways to form the outer fiber reinforcement of the devices of the invention it is possible to obtain devices of the invention with a unique combination of mechanical performance and osteoconductivity.

### MATERIALS

As mentioned earlier, the present invention relates to bioabsorbable materials and devices for musculoskeletal applications, such as e.g. bone fracture or osteotomy fixation, soft tissue (such as tendon) to bone fixation, soft tissue to soft tissue fixation and guided bone regeneration applications, such as vertebral fusion. Unlike other materials used in prior art, the composites of this invention have a continuous long fiber reinforcing, bioabsorbable coating phase surrounding at least one exterior surface of the core phase. The core phase can, however, also penetrate to the exterior surface, and the fiber reinforcement and the core can also be exposed on the same surface. The fiber reinforcement can also penetrate into the interior of the core. However, it is continuous and placed mainly on the exterior surface of the core. The reinforcing long fiber phase may comprise polymeric reinforcing fibers and optionally reinforcing ceramic or bioactive glass fibers. The matrix polymer component of the core can be, for example, any bioabsorbable or bioerodible polymer, copolymer, terpolymer or polymer alloy (mixture of two or more polymers or copolymers), and this matrix polymer component may also contain bioactive ceramic or glass filler or fibers. The polymer can be synthetic or "semisynthetic", which means polymers made by chemical modification of natural polymers (such as starch). Typical examples of polymers, which can be used in this invention, are listed in Table 1 below.

**TABLE 1. Bioabsorbable (resorbable) polymers, copolymers and terpolymers which may be applied to make devices of the invention (useful raw materials to manufacture bioabsorbable polymeric fibers and bioabsorbable polymeric core).**

| |
|---|
| Polyglycolide (PGA) |
| Copolymers of glycolide: Glycolide/L-lactide copolymers(PGA/PLLA) Glycolide/trimethylene carbonate copolymers (PGA/TMC) - Polylactides (PLA) |
| Stereocopolymers of PLA: Poly-L-lactide (PLLA) Poly-DL-lactide (PDLLA) L-lactide/DL-lactide copolymers |
| Other copolymers of PLA: Lactide/tetramethylglycolide copolymers Lactide/trimethylene carbonate copolymers Lactide/d-valerolactone copolymers Lactide/[epsilon]-caprolactone copolymers |
| Terpolymers of PLA: Lactide/glycolide/trimethylene carbonate terpolymers Lactide/glycolide/[epsilon]-caprolactone terpolymers PLA/polyethylene oxide copolymers |
| Polydepsipeptides |
| Unsymmetrically 3,6-substituted poly-1,4-dioxane-2,5-diones |
| Polyhydroxyalkanoates: Polyhydroxybutyrates (PHB), PHB/b-hydroxyvalerate copolymers (PHB/PHV) |
| Poly-b-hydroxypropionate (PHPA) |
| Poly-p-dioxanone (PDS) |
| Poly-d-valerolactone - Poly-e-caprolactone |
| Methylmethacrylate-N-vinyl pyrrolidone copolymers |
| Polyesteramides |
| Polyesters of oxalic acid |
| Polydihydropyrans - Polyalkyl-2-cyanoacrylates |
| Polyurethanes (PU) |
| Polyvinylalcohol (PVA) |
| Polypeptides |
| Poly-b-malic acid (PM LA) - Poly-b-alkanoic acids |
| Polycarbonates |
| Polyorthoesters |
| Polyphosphates |
| Polyanhydrides Tyrosine derived polycarbonates |

The continuous polymeric reinforcing fibers and possible ceramic or bioactive glass reinforcing fibers are recognizable and distinguishable in the final product. They may be distinguishable on the exterior surface of the final product, or they can be covered by matrix polymer or another coating and distinguishable only in the cross section of the destroyed final product.

The diameter of the reinforcing polymeric long fibers can vary typically between 4 µm and 800 µm, preferably between 20 µm and 500 µm. The most useful range is from 30 µm to 70 µm for multifilament bundles or yarns and from 70 µm to 500 µm for monofilaments. Useful polymers for the polymeric reinforcing fibers include several of those listed in Table 1, but the polymer has to be chosen so that at least part of the polymeric reinforcing fibers have a longer strength retention time *in vivo* than the polymeric matrix component has. The polymeric fibers can be used in the form of long single fibers, fiber bundles of one or more components, in the form of yarns, braids or bands, or in the form of different types of fabrics made by the methods of textile technology.

The bioactive element of the composite can be in the form of particulate fillers in the matrix or in the form of fibers used in conjugation with polymeric fiber reinforcement. Typical examples of bioactive elements suitable for use as particulate fillers are listed in Table 2.

The ceramic reinforcing fibers typically comprise biodegradable bioactive long (or short) fibers of bioactive glass with diameters typically from 1 µm to 800 µm and preferably from 5 µm to 500 µm.

Preferable diameters of ceramic reinforcing fibers are often in the range between 1 µm and 20 µm; especially the fibers with a diameter less than 10 µm can be of importance. Typical examples of materials suitable for use as ceramic or bioactive glass reinforcing fibers are also listed in Table 2. They can be used as short or long single fibers, as yarns, braids, bands or as different types of fabrics made by the methods of textile technology.

**TABLE 2. Bioceramics and glasses suitable for composites of the invention.**

| |
|---|
| Hydroxyapatite (HA) |
| Other calcium phosphates: such as Tricalcium phosphates (TCP) |
| Combinations of different calcium phosphates, such as HA/TCP |
| Calcium carbonate |
| Calcium sulphate |
| Bioactive glasses |
| Bioactive glass-ceramics |

Polymeric fibers and ceramic fibers may also be introduced into the polymer matrix or composite structure in the form of prefabricated products, such as prepregs, manufactured by techniques of the polymer composite technology in addition to the methods of textile technology.

The polymeric fibers of this invention are long and continuous, which means that the length of a substantial amount of fibers is preferably longer than or close to or equal to the circumference of the final product (device). Ceramic fibers are long fibers having a length at least 10 times their diameter. They are typically longer than 150 µm, preferably longer than 2 millimeters and more preferably longer than 30 millimeters. At their best, both the polymeric fibers and the possible ceramic fibers are continuous so that their length is equal to or greater than the circumference of the device. The fibers are longer than the circumference of the core, being continuous through the whole exterior surface of the device, and they encircle the core several times without any discontinuous point. If both the polymeric fibers and the ceramic fibers are used in conjugation as a fiber reinforcement, the length of the fibers can be further increased if the fibers are, e.g., twisted, wound or braided. The amount of the polymeric reinforcing fibers or ceramic reinforcing fibers in the composite is from 5 wt-% to 90 wt-%, preferably from 10 wt-% to 70 wt-%.

The matrix of the core for the devices of this invention can be composed of at least one bioabsorbable polymer, copolymer, terpolymer or polymer alloy, or a compound of polymer and bioactive ceramic or glass particulate filler (or short fibers filler/reinforcement). Bioactive filler acts as an osteoconductive bony ongrowth and ingrowth agent and provides a reservoir of calcium and phosphate ions, thus accelerating the bone healing. These ions may also have a buffering effect on the acidic degradation products of the resorbable polymeric components of the composite. While the matrix polymer degrades, bone can attach to the residual ceramic or glass material. Optional porosity in the polymer matrix can additionally facilitate the bone ingrowth (growing of bone inside of the core). The amount of bioactive ceramic or glass filler in the matrix is from 10 wt-% to 80 wt-%, preferably from 15 wt-% to 60 wt-%.

Accordingly, the bioactivity of the core can also be achieved by using ceramic or glass (short or long) fibers which also act as osteoconductive bioactive bony ongrowth and ingrowth agents, providing a reservoir of calcium and phosphate ions and accelerating the bone healing. In the same way as in materials in which bioactivity is achieved by using ceramic or glass filler in the matrix, these ions may also have a buffering effect on the acidic degradation products of the resorbable polymeric components of the composite. While the matrix polymer degrades, bone can attach to the residual ceramic or glass material. Optional porosity in the polymer matrix can accelerate the bone ingrowth process.

The bioactive ceramic or glass phase, independent of its form, may also increase the visibility of the devices in imaging systems, such as X-ray, MRI (magnetic resonance imaging), or CT (computed tomography). The visibility is, however, dependent on the ceramic or glass phase content of the composite device. Therefore, the bioactive ceramic phase can provide the composite with a radiopaque property, and it will not disturb radiographic images and does not make post surgical assessment of healing more difficult.

The materials of this invention may contain various additives and modifiers which improve the performance or processability of the device. Such additives include surface modifiers to improve the attachment between the polymeric and ceramic components. The devices may also contain pharmaceutically active agents, such as antibiotics, chemotherapeutic agents, wound-healing agents, growth hormones and anticoagulants (such as heparin). These agents are used to enhance the bioactive feature of the composite, to make it multifunctional and to improve the healing process of the operated tissues.

### MANUFACTURING

The manufacture of the composite can include any suitable processing methods of plastics technology, polymer composite technology and/or textile technology. The matrix polymer and the bioactive agent (bioceramic or bioactive glass and/or processing aids and/or any pharmaceuticals, such as antibiotics) can be mixed together by mechanical mixing, melt mixing or solvent mixing. The polymeric and/or ceramic reinforcing fibers can be used as plain fibers or in a modified form: for example, in the form of braided, knitted or woven to two- or three-dimensional structures (together or as separate fabrics) or in the form of preforms such as prepregs including a suitable bioabsorbable polymeric binding aid. The mixture of the matrix and the polymeric reinforcing fibers (and the ceramic reinforcing fibers) can be made by mixing, by coating or by using a solvent as an intermediate to preform the material (prepreg).The material preform or the final device can also be produced by various techniques including compression molding, transfer molding, filament winding, pultrusion, melt extrusion, mechanical machining or injection molding to any desired shape. Preferably, the core and the continuous fiber reinforcement are combined by means of a suitable molding method, such as compression molding, injection molding, filament winding, pultrusion, or ultrasonic molding.

In the manufacture of a medical device of the invention by compression molding, the polymeric long fiber reinforcement (fibers or prefabricated band-like preform made of the fibers) is reeled around the exterior surface of a core billet to form a continuous fiber reinforcement, being also able to penetrate into the interior of the core billet from its exterior surface. Thereafter, the fiber covered billet is placed into a compression molding mold and compressed to the desired shape at an increased temperature (above the Tg of the matrix polymer) and pressure. As the temperature rises above Tg of the matrix and compressive pressure is applied, the matrix polymer flows between the reinforcing fibers on the exterior surface of billet. The matrix polymer flow is facilitated if the reinforcing fiber bundle, prepreg or fabric contains open porosity or open spaces between fibers. Depending on the compression molding temperature and the chemical structure of the device components, the adhesion between the continuous fiber reinforcements and the core can be formed by secondary van der Waals forces (secondary chemical bonds) and/or by primary chemical bonds (e.g. there may be chemical bonds between the reinforcement and the matrix at their interfaces).

According to an advantageous embodiment, special features, such as holes, are made on the exterior surfaces of the final device during the compression molding without breaking the reinforcing fibers and thus keeping the reinforcement continuous. This can be done by using protruding inserts in the compression molding mold cavity to create the desired features by penetrating through the exterior surface of the device billet before or during the compression molding process. The core billet can be designed so that the reinforcing long fibers dodge on the outer surface of the protruding insert; therefore, no cut discontinuity is created in the reinforcement. Protruding inserts can also be used when the matrix is heated above the Tg of the matrix and some of the reinforcing fibers are pressed from the exterior surface of the device billet to the inside of the matrix, creating special features without breaking the continuity of the fiber reinforcement.

If injection molding is used, the polymeric long fiber reinforcement can be used as a preprocessed product, such as a knitted fabric or a braid, which is in a form of a continuous ring-like or tube-like structure and is placed inside the injection molding mold chamber. The matrix is then introduced into the chamber, e.g., from the middle of the chamber on its outer wall, so that the reinforcing fiber fabric is forced to stay in contact with the outer wall of the inside of the mold cavity. In the same way as in compression molding, specially designed protruding inserts can be used in injection molding to create special features, such as holes and cavities, on the exterior surface of the final product, without breaking the long fibers.

If the filament winding or pultrusion technique is used, the reinforcing fibers are reeled around a mandrel, which is composed of a combination of at least one bioabsorbable polymer and a bioactive filler, the mandrel forming the core of the end product.

When the polymeric and/or ceramic long reinforcement fibers of the devices of the invention are continuous, the devices have better mechanical properties than short or non-continuous long fiber reinforced bioabsorbable devices. One of the most important factors is thus the absence of fiber ends in the continuous fiber reinforced devices, which fiber ends can be sites for crack initiation during fracture due to mechanical loading.

Processing methods for manufacturing of fiber reinforcement composed of both ceramic and polymeric reinforcing fibers are disclosed e.g. in WO2006114483.

The fiber reinforced bioactive composite materials and devices described in this invention have improved mechanical properties when compared to non-reinforced devices, because the fiber reinforcement changes the behavior of the materials and thus makes the reinforced device stronger and more reliable under loading and also more reliable if the implant develops a fracture (or fractures). This feature is very important for load bearing applications, such as spinal fusion and bone fracture fixation applications.

The fiber orientation can vary in different embodiments of this invention. The reinforcing fibers can be parallel or they can be stacked to two or more layers with different angles between different layers. A random orientation is also possible.

The core of the composite of the invention may be composed of laminated layers which, in addition to the continuous fiber reinforcement on at least one exterior surface, can also contain reinforcing fibers. The core in the middle of the implant structure may be composed of layers of the laminate which are laminated (stacked) together by using heat and pressure. Those laminated layers form the core and the interior of the device, and they may also contain reinforcing fibers which are similar to those used on the outer surface of the device, which surface is covered by a continuous fiber reinforcement. The number of the layers to be laminated together varies depending on the desired end use. Such laminated structures are useful, for example, in surgical fixation devices, such as fixation plates for bone fractures, or in spinal fusion devices. The fiber orientation in the superimposed layers of the device may differ from layer to layer. In such a manner, it is possible to manufacture devices having a very strong and tough exterior surface.

Composite samples, such as rods, tubes and plates, can be applied as such as devices (implants) for tissue fixation, regeneration or tissue generation. The composite samples can also be processed further mechanically and/or thermally into the form of more sophisticated devices, e.g. screws, plates, nails, tacks, suture anchors, bolts, clamps, wedges, cages, etc., to be applied in different disciplines of surgery for tissue management, such as tissue fixation (e.g. bone to bone fixation, soft tissue to bone fixation, and soft tissue to soft tissue fixation), or to help or guide tissue regeneration and/or generation.

In the following, the subject matter of this application is explained by examples and by referring to figures in which
- Fig. 1: shows cross-sectional views of medical devices,
- Figs. 2a to 2f: show sections of the core and the medical device in the longitudinal direction,
- Fig. 3: shows core billet designs,
- Fig. 4: shows continuous fibers on the exterior surface of the core billet,
- Fig. 5: shows a core billet (Fig. 4a) and a reinforced core (Fig. 4b),
- Fig. 6: shows the arrangement of the hole tearing test to evaluate the effect of continuous fiber reinforcement on the outer cylindrical surface of the core, and
- Figs. 7 to 9: show typical hole tearing test results for fiber reinforced and non-reinforced implants.

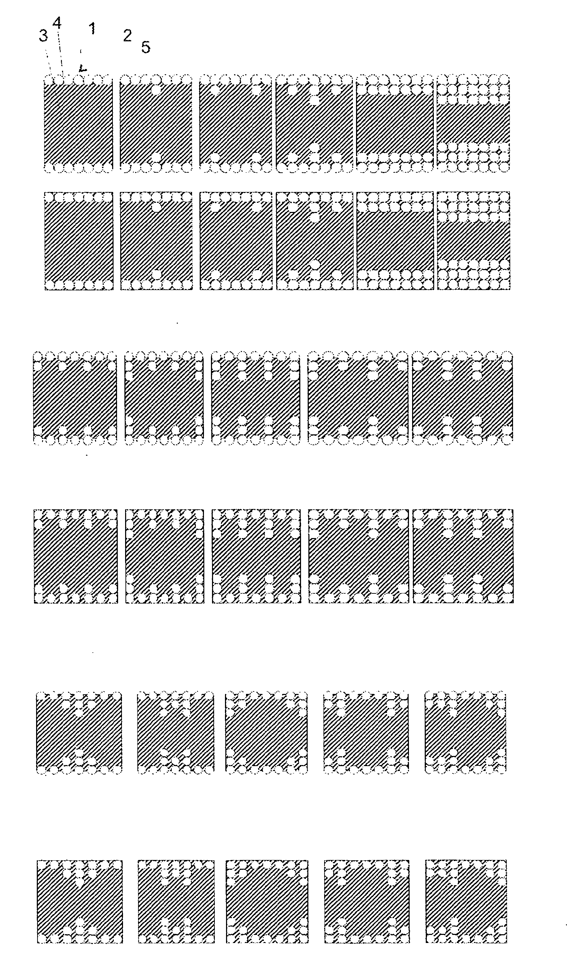

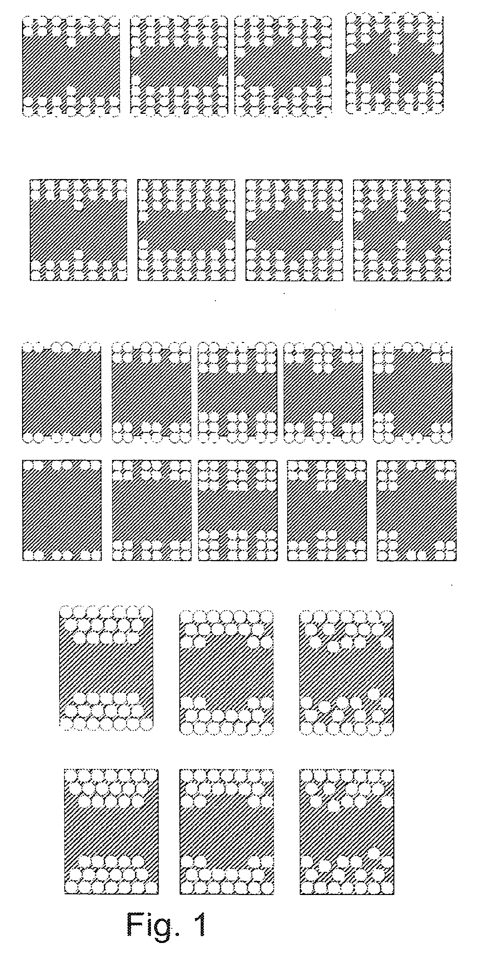

Fig. 1 shows cross-sectional views of medical devices 1 which are reinforced with a bioabsorbable structure 2. In this case the bioabsorbable structure is a monofilament fiber which is wound around a core 3. Before the monofilament fiber is wound around the core 3, the outer wall 4 may be provided with grooves 5.

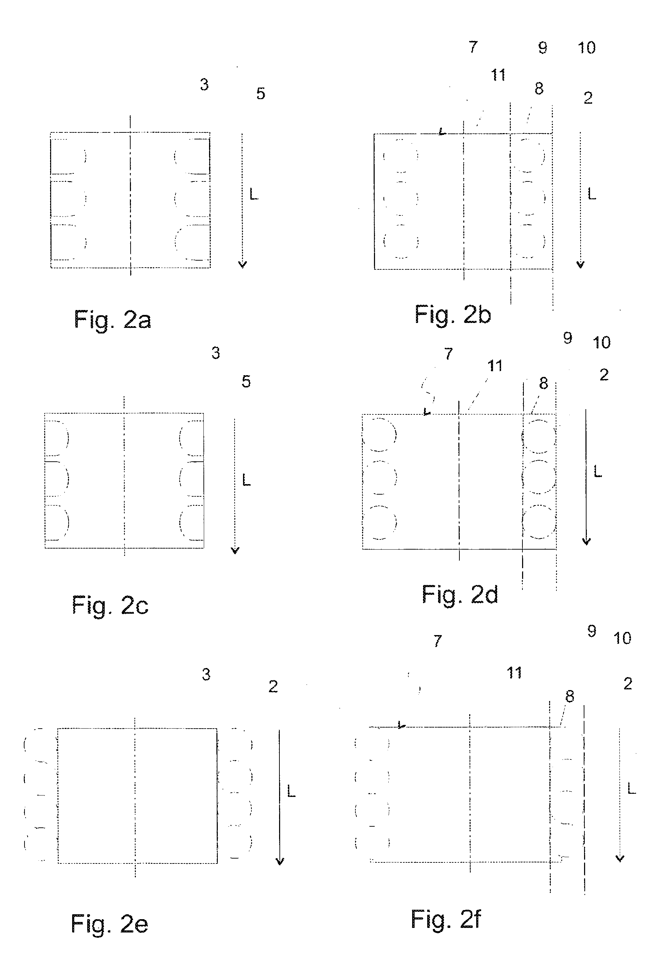

Fig. 2 shows lengthwise sections of the core 3 and the body 7. Fig. 2a shows a core 3 with prefabricated grooves 5 in which the bioabsorbable reinforcing structure 2, in this case a monofilament fiber, is to be placed. The depth of the grooves is approximately equal to the diameter of the monofilament fiber. Fig. 2b shows the structure of the body 7 after the reinforcing structure 2 has been inserted in the grooves 5 of the core 3 and the core 3 with the reinforcing structure has been treated in the subsequent process step, such as compression molding. The fiber has been left inside the material of the core so that the bioabsorbable basic material covers the fiber. In Fig. 2b the peripheral region 8 is between the dashed lines 9 and 10.

Fig. 2c shows another core 3 with prefabricated grooves 5 which are more shallow than in Fig. 2a so that the reinforcing structure 2 will protrude from the core 3 when a monofilament fiber having the same diameter as in Fig. 2b is placed in the grooves 5. Fig. 2d shows the structure of the body 7 after the reinforcing structure 2 has been inserted to the grooves 5 of the core 3 and the core 3 with the reinforcing structure 2 has been treated in the subsequent process step, such as compression molding. The reinforcing fiber contacts the outer surface of the medical device, i.e. the width of the peripheral region corresponds approximately to the diameter of the fiber. In Fig. 2d the peripheral region 8 is between the dashed lines 9 and 10.

Fig. 2e shows yet another core 3 which has no prefabricated grooves but the reinforcing fiber is wound around the core. Fig. 2f shows the structure of the body 7 after the reinforcing structure 2 has been inserted in the grooves 5 of the core 3 and the core 3 with the reinforcing structure 2 has been treated in the subsequent process step, such as compression molding. As can be seen from Fig. 2f, the reinforcing fiber mainly forms the outer surface of the body, i.e. the peripheral region 8 of the medical device extends further than the outer edge of the core 3. In Fig. 2f the peripheral region 8 is between the dashed lines 9 and 10.

### Example 1.

### Manufacturing of implant (device) prototypes

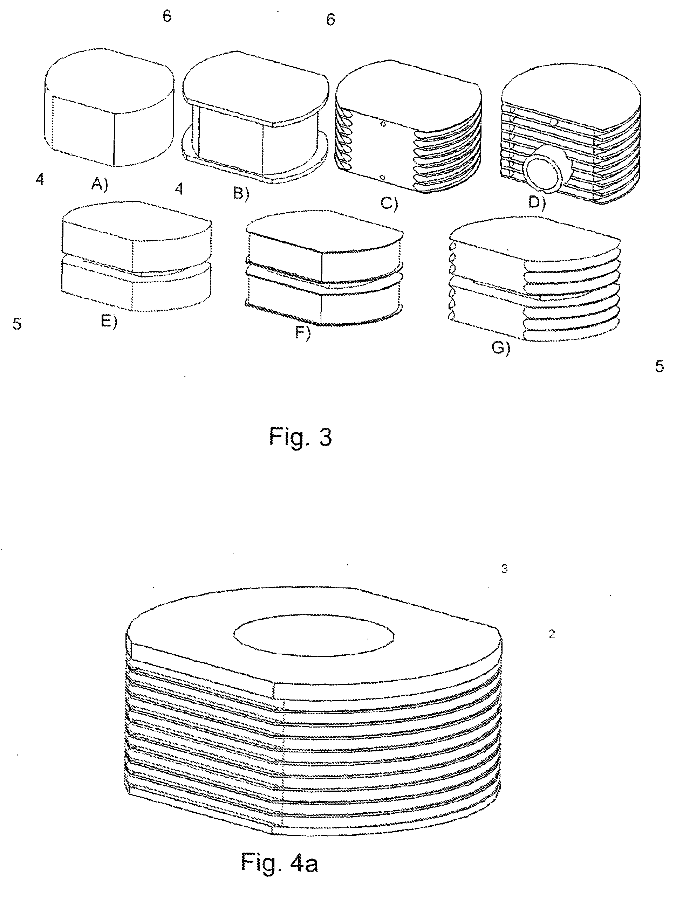

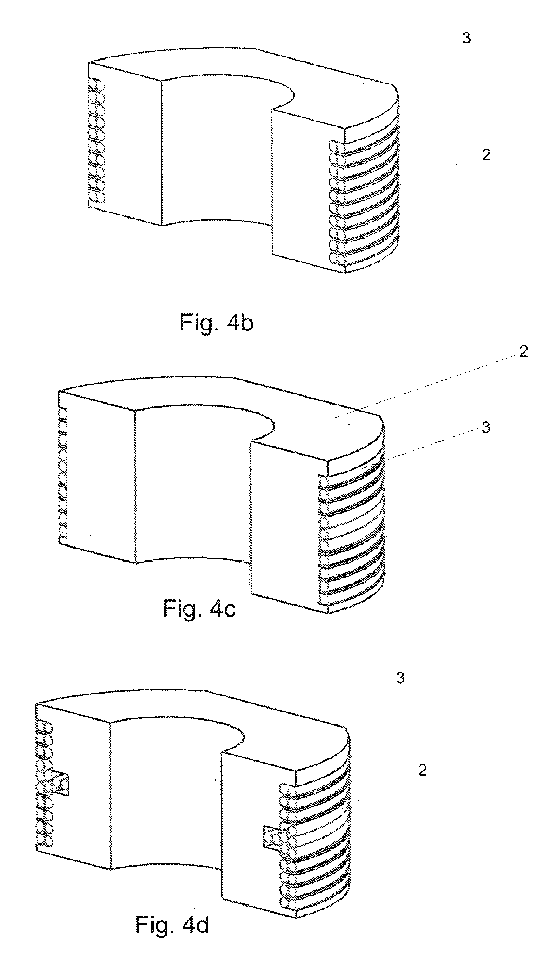

A cylindrical long billet (a bar with a diameter of about 15 mm, IV 4.0) was melt extruded from a powder mixture of 50 wt-% of poly-L/DL-lactide 70/30 (IV 6.13, Boehringer Ingelheim) and 50 wt-% of β-tricalcium phosphate (50 wt-% 125 µm granules, Plasma Biotal). Reinforcing fibers (IV ca. 3.6) were manufactured with a twin screw extruder from poly-L/D-lactide 96/4 (IV 5.17, Purac Biochem). No organic or inorganic solvents or any processing additives were used in the manufacturing process. After the extrusion, the bar was machined manually into various forms of billets (cores of devices of the invention) having a smooth exterior surface or having various guiding grooves for fibers on their exterior surface. Schematic figures of the options of some of the core designs are given in Figure 3. As one can see from Fig. 3, there are a lot of variations concerning the outer wall 4 and the end walls 6. The exterior smooth surfaces and grooves were filled by several circles of continuous fibers as is seen in Figure 4. There may be more than one fiber layer in the groove, as shown in Figs. 4b and 4d, or only one fiber layer in the groove, as shown in Fig. 4c. The fibers were located on the smooth outer surface or in the grooves on the outer surface of the core billet. After that, the fiber covered cores were placed into a compression molding mold (height 4-10 mm, diameter of cylindrical part 16.3 mm and length 13.7 mm). The mold was subjected to compression and an increased temperature (time 1-30 min, temperature 130-145°C, compressive force 1-20 kN). Implant prototypes were ejected from the mold after cooling the mold to room temperature or a lower temperature, and after that, a hole was drilled in the middle of each device manually.

At lower temperatures (below 140°C), the bonding between the fibers and the matrix was more mechanical than chemical. The mechanical bonding was, however, increased by using grooved core billets.

At higher temperatures (140°C-145°C), the bonding was a combination of mechanical and chemical. Again, the mechanical bonding was increased by using grooved core billets. The higher the temperature, the higher the chemical bonding.

### Example 2.

### Manufacturing of implant (device) prototypes with threaded instrument hole on one surface.

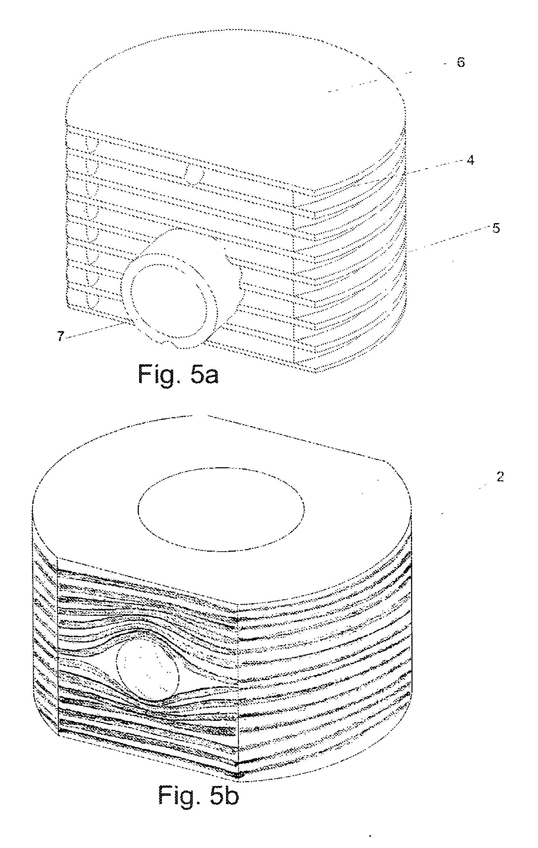

Implant prototypes were manufactured in the same way as in Example 1, using the same raw materials. A 3 mm threaded hole (M3) for an implantation instrument was made during the compression molding process by using a kernel which protruded into a pre-machined hole in the core billet. The core billet used with the pre-machined hole 7 for the kernel is shown in Figure 5.

Figure 5a shows the core billet used with a pre-machined hole for a kernel and surrounding grooves for reinforcing fibers. Fibers are wound around the core billet so that they dodge the protrusion on the front side of the billet.

Figure 5b shows a composite manufactured from the core billet presented in Figure 5a. Fibers (shown in black) dodge the threaded hole on the front side of the composite.

The aim of this example was to show the feasibility of manufacturing implants with protruding features, such as holes, and a continuous fiber reinforcement on the exterior surface of the device.

### Example 3.

Two different types of implant prototypes were manufactured by the technique presented in Example 1. The core of the implant prototypes was composed of 50 wt-% of poly-L/DL-lactide 70/30 (IV 6.13 Boehringer Ingelheim) and 50 wt-% of β-tricalcium phosphate granules (Plasma Biotal) (I.V of the polymer matrix after extrusion was about 4.0). The fiber reinforcement was composed of poly-L/D-lactide 96/4 (IV 5.17, PURAC Biochem) which was processed into the form of monofilament fibers (I.V. about 3.6 after extrusion, diameter 360-430 µm).

Two types of implants (see Figure 1A and 1C) were manufactured: (a) non-reinforced (prior art) specimens composed of pure core ("50/50 sample") and (b) continuously and circumferentially fiber reinforced specimens, in which the core of (a) was surrounded by a continuous poly-L/D-lactide 96/4 fiber reinforcement ("50/50 + fibers-sample"). A core having round grooves for fibers (see Figure 1 C) was used. The fiber reinforcement was reeled around the grooved core without any solvents or additives.

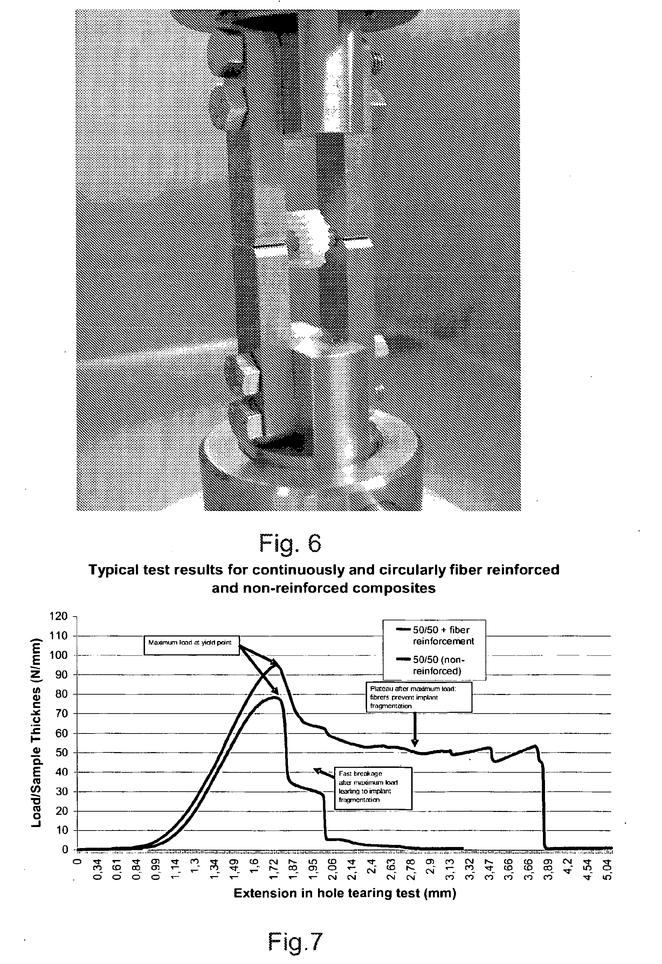

A hole tearing test was made using custom made jigs and a Lloyd 2000S testing machine. The test speed was 5 mm/min. Prototype implants were placed in testing jigs as is shown in figure 6. The aim of the hole tearing test was to evaluate the reinforcing effect of continuous and circular fiber reinforcement on the outer surface of the core compared to the non-reinforced core. Results of the hole tearing test are shown in Figure 7 and in Table 4. Because the examined composites did not have an identical height, the load/sample height ratio was analyzed to make the examined composites comparable.

It can be seen from Figure 7 and Table 4 that the fiber reinforcement increased the maximum tear load by 16% when the thickness of the implant was taken into account, when comparing to the respective values of non-reinforced cores. Another important finding was that the fiber reinforcement changed the fracturing mechanism (the fragmentation to pieces by tear) of the implants. In the case of non-reinforced specimens, implant fragmentation to pieces by tear occured at a displacement of about 2 mm, but in the case of fiber reinforced specimens, the fragmentation to pieces occurred only after a displacement of 3.8 mm. This delaying of fragmentation can be seen as a plateau between 300-400 N after the maximum load in the case of the fiber reinforced specimens in Figure 7.

In clinical applications, the delayed fragmentation is an additional safety factor, because implant samples can migrate in tissues (with possible adverse effects) only after fragmentation.

**Table 4. Comparison of hole tearing test results for fiber reinforced and non-reinforced implants.**

| **Sample** | **Maximum Load (N)** | **Sample height (mm)** | **Maximum Load /sample height** |
|---|---|---|---|
| **50/50** | 438,7 | 5,6 | 77,9 |
| **50/50 + fibers** | 557,7 | 6,2 | 90,4 |
| **Effect of fiber reinforcement** | | | +16 % |

### Example 4.

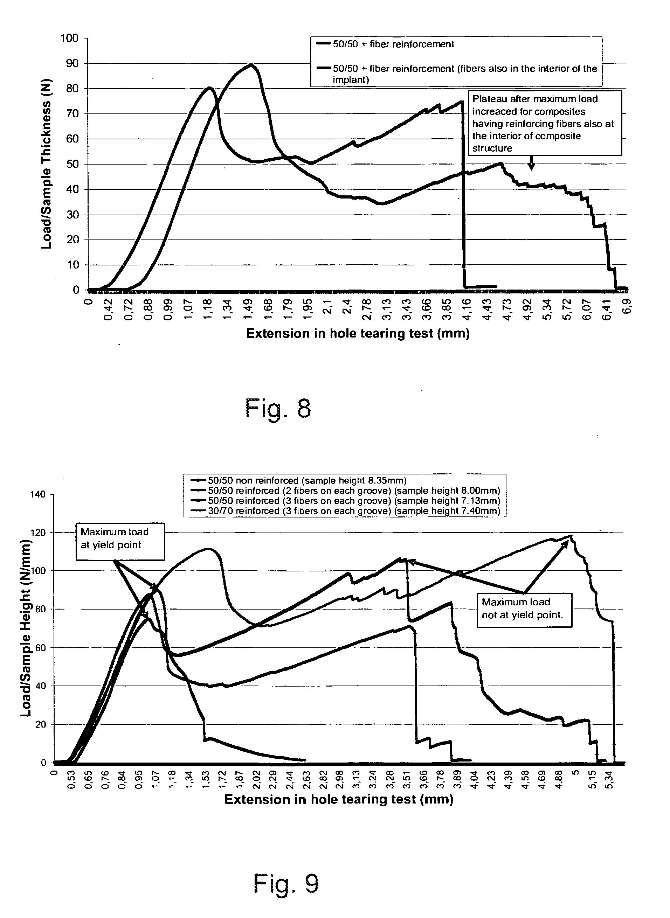

Implant prototypes were manufactured from the same raw materials in the same way as in example 3. The only difference was the design of the core billets, as one batch of billets had round grooves on the exterior surface of the billet (50/50 + fiber reinforcement) (Figure 1 C) and the other batch had a deep groove in the exterior surface penetration into the interior of the core in addition to round grooves in the exterior surface (50/50 + fiber reinforcement (fibers also in the interior of the implant structure)) (Figure 1 G). Hole tearing tests were made identically to Example 3. The results are shown in Figure 8 and in Table 5.

**Table 5. Comparison of hole tearing test results for fiber reinforced implant prototypes with different designs of fiber reinforcement.**

| **Sample** | **Maximum Load (N)** | **Sample height (mm)** | **Maximum load /sample height** |
|---|---|---|---|
| **50/50 + fibers** | 486,0 | 6,0 | 80,6 |
| **50/50 + fibers and deep groove** | 558,9 | 6,4 | 88,0 |
| **Effect of fiber reinforcement** | | | + 9% |

It can be seen from Figure 8 and Table 5 that the design of the fiber reinforcement affected the maximum load and the fracturing mechanism. When the fiber reinforcement was also present in the interior of the implant, the maximum load increased by 9% when the thickness of the implant prototype was taken into account, in comparison with the implant prototypes having fiber reinforcement on the exterior surface only. The fracturing mechanism was also changed, while the implant fragmentation was delayed more when fibers were also present in the interior of the composite.

### Example 5

Composites with a 3 mm threaded hole (M3) on the exterior surface were manufactured according to Example 2 from the same raw materials as presented in Example 1. The only difference in the raw materials was that in addition to composites containing 50 wt-% of β-TCP, also 30 wt-% of β-TCP containing composites were manufactured. The core billet design for the fiber reinforced specimens is presented in Figure 1 G. The fiber reinforcement dodged the treaded hole as presented in Figure 4b in Example 2. For non-reinforced specimens (50/50), a core billet (Figure 1A) with a pre-machined hole for a kernel was used. The testing of the composites was identical to that of Examples 3 and 4. The test results are shown in Figure 9 and in Table 6.

**Table 6. Comparison of hole tearing test results for fiber reinforced implant prototypes with different designs of fiber reinforcement.**

| **Sample** | **Load at yield (N)** | **Maximum Load (N)** | **Sample height (mm)** | **Load at yield /sample height** | **Maximum load /sample height** | **Effect of fiber reinforcement compared to 50/50 non reinforced (Load at Yield)** | **Effect of fiber reinforcement compared to 50/50 non reinforced (Maximum Load)** |
|---|---|---|---|---|---|---|---|
| **50/50 non reinforced** | 607,7 | 607,7 | 8,03 | 75,8 | 75,8 | - | - |
| **50/50 + fiber reinforcement (2 fibers on each groove)** | 690,5 | 690,5 | 7,85 | 88,0 | 88,0 | 16,1 | 16,1 |
| **50/50 + fiber reinforcement (3 fibers on each groove)** | 607,3 | 749,6 | 7,1 | 85,5 | 105,6 | 12,9 | 39,4 |
| **30/70 + fiber reinforcement (3 reinforcement (3 fibers on each groove)** | 825,5 | 875,1 | 7,4 | 111,6 | 118,3 | 47,3 | 56,1 |

It can be seen from Figure 9 and Table 6 that both the amount of reinforcing fibers in the composite and the composition of the matrix affected the maximum load and the fracturing mechanism. When there were 2 circles of fibers on each groove of the matrix billet, the load at yield point was the maximum load, but when there were 3 circles of fibers on each groove, the maximum load was reached several millimeters after the yield point. In any case, the fiber reinforcement increased the breakage of the matrix (the load at yield point increased by 12.8 - 47.2% when compared to 50/50 non reinforced medical device), but when there were 3 circles of fibers on each groove, it could be seen that the composites had an even higher resistance to tear force than the reinforced matrix had. Therefore, the implant fragmentation could be prevented efficiently by increasing the fiber content. There was no remarkable difference in the load at yield between the composites having 2 or 3 fibers in each groove, but composites with 3 circles of fibers in each groove had a much higher maximum load. For the specimens containing 3 rounds circles of reinforcing fibers on each groove, the maximum load/sample thickness ratio increased by 39.3% for 50 wt-% β-TCP containing specimens and by 56.1 % for 30 wt-% β-TCP containing specimens when compared to non-reinforced 50/50 structures.

In the same way as in Example 4, also here the increase in the fiber content raised the point of fragmentation into pieces (i.e. the plateau state increased remarkably).

### Example 6

A tubular billet having an outer diameter of 5 mm and an inner hole diameter of 2,5 mm can be extruded from 80L/20G PLGA mixing 25 w-% of HA powder into the structure using a twin screw extruder equipped with a suitable tube die. The billet can be cut to suitable length (like 30 mm long) pieces and covered with three layers of 0,3 mm thick, continuous 96L/4D PLA fiber by filament winding method, to make a preform having the polymer ceramic composite tube in the centre and the continuous reinforcing fiber around the structure. This preform can be placed into a compression moulding mould having on the inside surface the desired external form of an ACL-screw (ACL = Anterior Crucial Ligament) and a parting surface along the longitudinal axis of the screw. The mould can be open from one end, with a circular opening. A piston having the form of the desired instrumentation, which can be used in the implantation of an ACL-screw, can be pushed into the open channel of the mould and can be used as a plunger in compression moulding of the screw at 140°C. After cooling down the mold the compression force can be relieved and the piston can be pulled out from the mould. The mould can be opened along it's parting surface and an ACL-screw can be ejected from the mould. The composite ACL-screw will have a high percentage of the osteoconductive filler material in its structure, but excellent resistance against the breakage during the insertion due to the circumferential fiber reinforcing structure.

## Claims

1. A medical device for surgical tissue fixation or guided tissue regeneration and/or generation comprising a body (7) comprising bioabsorbable basic material and having a longitudinal axis (L), the body (7) comprising an inner region (11) and a peripheral region (8) transverse to the longitudinal axis (L) so that the peripheral region (8) surrounds the inner region (11), the inner region (11) being made of bioabsorbable basic material and the peripheral region (8) comprising the bioabsorbable basic material, **characterized in that** the peripheral region (8) comprises a bioabsorbable reinforcing structure (2) wound around the inner region, in addition to the bioabsorbable basic material.

2. The medical device according to claim 1, **characterized in that** the bioabsorbable reinforcing structure (2) comprises long, continuous bioabsorbable polymeric fibers, whose length exceeds or is equal to the length of the circumference of a core of the fibers.

3. The medical device according to claim 1 or 2, **characterized in that** the bioabsorbable reinforcing structure (2) comprises a monofilament in the peripheral region (8) of the body (7) and the monofilament advances in parallel to the circumference of the body (7) around the body (7).

4. The medical device according to claim 1 or 2, **characterized in that** the bioabsorbable reinforcing structure (2) comprises a yarn manufactured from staple fibers and the yarn advances in parallel to the circumference of the body (7) around the body (7).

5. The medical device according to any preceding claim, **characterized in that** the bioabsorbable reinforcing structure (2) comprises ceramic or bioactive glass fibers.

6. The medical device according to any preceding claim, **characterized in that** the bioabsorbable basic material comprises copolymer poly-L/DL-lactide and β-tricalcium phosphate.

7. The medical device according to any preceding claim, **characterized in that** the bioabsorbable structure comprises copolymer poly-L/D-lactide.

## Patentansprüche

1. Medizinische Vorrichtung zur chirurgischen Gewebefixierung oder gesteuerten Geweberegeneration und/oder -generation umfassend einen Körper (7), der ein bioabsorbierbares Grundmaterial umfasst und eine Längsachse (L) aufweist, wobei der Körper (7) einen Innenbereich (11) und einen Umfangsbereich (8) quer zur Längsachse (L) derart umfasst, dass der Umfangsbereich (8) den Innenbereich (11) umgibt, wobei der Innenbereich (11) aus bioabsorbierbarem Grundmaterial besteht und der Umfangsbereich (8) das bioabsorbierbare Grundmaterial umfasst, **dadurch gekennzeichnet, dass** der Umfangsbereich (8) zusätzlich zu dem bioabsorbierbaren Grundmaterial eine rings um den Innenbereich gewickelte bioabsorbierbare Verstärkungsstruktur (2) umfasst.

2. Medizinische Vorrichtung nach Anspruch 1, **dadurch gekennzeichnet, dass** die bioabsorbierbare Verstärkungsstruktur (2) lange, durchgehende bioabsorbierbare Polymerfasern umfasst, deren Länge die Länge des Umfangs eines Kerns der Fasern überschreitet oder dieser gleicht.

3. Medizinische Vorrichtung nach Anspruch 1 oder 2, **dadurch gekennzeichnet, dass** die bioabsorbierbare Verstärkungsstruktur (2) einen Einzelfaden im Umfangsbereich (8) des Körpers (7) umfasst und der Einzelfaden parallel zum Umfang des Körpers (7) rings um den Körper (7) geführt ist.

4. Medizinische Vorrichtung nach Anspruch 1 oder 2, **dadurch gekennzeichnet, dass** die bioabsorbierbare Verstärkungsstruktur (2) ein aus Stapelfasern hergestelltes Garn umfasst und das Garn parallel zum Umfang des Körpers (7) rings um den Körper (7) geführt ist.

5. Medizinische Vorrichtung nach irgendeinem vorangehenden Anspruch, **dadurch gekennzeichnet, dass** die bioabsorbierbare Verstärkungsstruktur (2) Keramikfasern oder bioaktive Glasfasern umfasst.

6. Medizinische Vorrichtung nach irgendeinem vorangehenden Anspruch, **dadurch gekennzeichnet, dass** das bioabsorbierbare Grundmaterial das Copolymer Poly-L/DL-lactid und β-Tricalciumphosphat umfasst.

7. Medizinische Vorrichtung nach irgendeinem vorangehenden Anspruch, **dadurch gekennzeichnet, dass** die bioabsorbierbare Struktur das Copolymer Poly-L/D-lactid umfasst.

## Revendications

1. Dispositif médical pour la fixation du tissu chirurgical ou la régénération et/ou la génération guidée du tissu comprenant un corps (7) comprenant un matériau bioabsorbable de base et ayant un axe longitudinal (L), le corps (7) comprenant une région interne (11) et une région périphérique (8) transversale par rapport à l'axe longitudinal (L) de sorte que la région périphérique (8) entoure la région interne (11), la région interne (11) étant constituée de matériau bioabsorbable de base et la région périphérique (8) comprenant le matériau bioabsorbable de base, **caractérisé en ce que** la région périphérique (8) comprend une structure bioabsorbable de renfort (2) enroulée autour de la région interne, en plus du matériau bioabsorbable de base.

2. Dispositif médical selon la revendication 1, **caractérisé en ce que** la structure bioabsorbable de renfort (2) comprend des fibres polymères longues, continues bioabsorbables, dont la longueur excède ou est égale à la longueur de la circonférence d'un noyau des fibres.

3. Dispositif médical selon la revendication 1 ou 2, **caractérisé en ce que** la structure bioabsorbable de renfort (2) comprend un monofilament dans la région périphérique (8) du corps (7) et le monofilament avance parallèlement à la circonférence du corps (7) autour du corps (7).

4. Dispositif médical selon la revendication 1 ou 2, **caractérisé en ce que** la structure bioabsorbable de renfort (2) comprend un fil fabriqué à partir de fibres discontinues et le fil avance parallèlement à la circonférence du corps (7) autour du corps (7).

5. Dispositif médical selon l'une quelconque des revendications précédentes, **caractérisé en ce que** la structure bioabsorbable de renfort (2) comprend des fibres céramiques ou bioactives en verre.

6. Dispositif médical selon l'une quelconque des revendications précédentes, **caractérisé en ce que** le matériau bioabsorbable de base comprend un copolymère de poly-L/DL-lactide et de phosphate β-tricalcique.

7. Dispositif médical selon l'une quelconque des revendications précédentes, **caractérisé en ce que** la structure bioabsorbable comprend un copolymère de poly-L/D-lactide.
